# EUROPEAN PATENT APPLICATION

(11) **EP 4 656 123 A1**
(43) Date of publication of application: **03.12.2025**
(21) Application number: 24178976.7
(22) Date of filing: 30.05.2024
(51) Int. Cl.: A61B 5/00, A61B 7/00, A61F 5/56, A61M 16/00, G16H 40/67, A61B 5/087

(54) **A METHOD FOR SNORE DETECTION IN A SLEEP APPLIANCE AND SLEEP APPLIANCE**

(71) Applicant: Koninklijke Philips N.V., 5656 AG Eindhoven (NL)
(72) Inventor: DUINEVELD, Paulus Cornelis, Eindhoven (NL); BOONSTRA, Bonne Lambert, Eindhoven (NL)
(74) Representative: Philips Intellectual Property & Standards

(57) **Abstract**

A method (50) for snore detection in a sleep appliance (4) comprises executing an app on a mobile device (34) and operating the sleep appliance for delivery of a pressurized flow of breathable gas to a patient interface device (14). The app is configured to: (i) record, via a microphone (42) of the mobile device, acoustic signals during use of the sleep appliance, (ii) analyze, via a processor (24,42) of at least one of the mobile device, the sleep appliance, or a remote cloud device (36), the recorded acoustic signals for generating processed audio data to detect snoring, and (iii) link, via a communication means (32,44), at least one of the recorded acoustic signals, the analyzed acoustic signals, and the recognized snoring characteristics or events between (a) the mobile device (34) or the remote cloud device (36), and (b) the sleep appliance (4). Analyzing includes using an acoustic model configured to recognize snoring characteristics or events by inspecting transient acoustic pressure levels and/or a frequency spectrum in the recorded acoustic signals.

## Description

### BACKGROUND

The present embodiments relate generally to sleep appliances and more particularly, to a method for snore detection in a sleep appliance and the sleep appliance thereof.

In a sleep or respiratory device, it is important to detect snoring, as snoring could be a sign that the mask pressure is too low. Snore detection is currently done with the pressure sensor in the sleep appliance device which its primary function is to control the mask pressure. Snoring is detected as a fluctuation of the pressure level in a certain frequency bandwidth (typically between 50-150 Hz).

The disadvantage of the current method is the signal to noise ratio. The pressure sensor is selected to accurately measure pressures up to 4-25 cm of water, i.e. 400-2500 Pa, while sound pressure levels are typically orders of magnitude lower. For instance, snoring at 80 dB sound pressure level will generate a pressure variation of the order of 0.5 Pa, which is small compared to the full scale of the pressure sensor and is therefore very challenging to detect. This is mainly due to the pressure sensor being inside the sleep appliance, which will further reduce the pressure variation due to snoring reaching the pressure sensor. Ideally one wants to detect less loud snoring signals which are closer to the 60 dB, which is already a decade lower absolute pressure. These pressure amplitudes become comparable to what a blower in the sleep appliance is producing. The blower noise can vary from one fan to another fan and is also very dependent on the air flow and mask pressure. Air flow and pressure generated by the blower is a function of the breathing pattern (i.e., at inhalation there is more air flow compared to exhalation).

Therefore, snore detection via the pressure sensor in the known sleep appliance is complicated. Accordingly, an improved method and apparatus for overcoming the problems in the art is desired.

### SUMMARY

According to one embodiment of the present disclosure, a method for snore detection in a sleep or respiratory appliance comprises executing an app on a mobile device and operating the sleep or respiratory appliance for delivery of a pressurized flow of breathable gas to a patient interface device to be worn by a user of the sleep or respiratory appliance. The app is configured to: (i) record, via a microphone of the mobile device, acoustic signals during a period of use of the sleep or respiratory appliance by a user during a period of sleep, (ii) analyze, via a processor of at least one of the mobile device, the sleep or respiratory appliance, or a remote cloud device, the recorded acoustic signals for generating processed audio data to detect snoring, wherein analyzing the recorded acoustic signals includes at least using an acoustic model configured to recognize snoring characteristics or events by inspecting transient acoustic pressure levels and/or a frequency spectrum in the recorded acoustic signals, and (iii) link, via a communication means, at least one or more of the recorded acoustic signals, the analyzed acoustic signals, and the recognized snoring characteristics or events between (a) at least one of the mobile device or the remote cloud device, and (b) the sleep or respiratory appliance. Operating the sleep or respiratory appliance includes snoring detection based on a combination of (i) sensor information obtained from sensors of the sleep or respiratory appliance and (ii) the processed audio data which is used to detect snoring. Operating further includes generating quantitative snoring information. In addition, the frequency spectrum in the recorded acoustic signals may include a frequency range selected from the group consisting of 10 to 3000 Hz, 25 to 500 Hz, and 50 to 150 Hz.

According to another embodiment, the method comprises wherein snoring detection includes combining, via the app alone, the sleep or respiratory appliance alone, the remote cloud device alone, or a combination of the app, the sleep or respiratory appliance, and the remote cloud device (i) the sensor information obtained from sensors of the sleep or respiratory appliance and (ii) data handling of processed audio data from the mobile device. With respect to sensor information, the sensor information is obtained from sensors of the sleep or respiratory appliance for determination of (i)(a) a moment or start of inspiration or (i)(b) a moment or start of expiration. With respect to data handling, the processed audio data from the mobile device is used (ii)(a) for detecting a noise spectrum at a start of a sleep procedure that includes providing the flow of breathable gas to the patient interface device, prior to or before a user begins snoring and (ii)(b) for detecting snoring via a sound level in the processed audio data above a snoring sound minimum level threshold and one or more of the moments or starts of inspiration and expiration.

In another embodiment, the method comprises wherein generating quantitative diagnostic snoring information is based on the detected snoring, wherein the quantitative diagnostic snoring information comprises an improved snoring information over similar snoring information based on use of the sleep or respiratory appliance alone. In yet another embodiment, snoring detection further comprises time synchronization of the recorded acoustic signals from the mobile device with the sleep or respiratory appliance sensor information relating the recorded acoustic information with phase information of a breathing cycle of the user.

In a further embodiment, the method includes wherein snoring detection further comprises (i) detecting (a) a moment (i.e., time of occurrence) of rise in a level of sound captured or recorded via the microphone of the mobile device (i.e., processed audio data) above a snoring sound minimum level threshold (also above background noise) that correlates with (i.e., correlating with, matching, or equaling) either one or a combination of both (b)(1) the moment of inhalation (i.e., determined via sleep appliance sensor signals signaling inhalation) and (b)(2) the moment of exhalation (i.e., determined via sleep appliance sensor signals signaling exhalation) within a snoring sound detection time threshold, and (ii) otherwise not detecting. In one embodiment, the snoring sound detection time threshold comprises within a time of ± 0.4 seconds. Determining the moment of rise in the level of sound captured or recorded via the microphone of the mobile device (i.e., processed audio data) includes background noise filtering by subtracting a background noise level of the noise spectrum prior to determining the rise above the snoring sound minimum level threshold. The background noise level of the noise spectrum may be due to the sleep appliance, determined from the level of sound captured or recorded via the microphone of the mobile device (i.e., in the processed audio data).

In accordance with another embodiment, the app is initiated via at least one of (i) a user input on the mobile device to start the app, and (ii) a sleep appliance routine. The sleep appliance routine is configured to wirelessly search and connect with the mobile device, wherein, responsive to being wirelessly connected with the mobile device, the sleep appliance routine (a) automatically starts the app or (b) causes a user inquiry requesting user input on the mobile device for confirmation to start the app. In yet another embodiment, a sound level threshold of the recorded acoustic signals via the microphone of the mobile device is in a range of 5-20 dB larger than, or in a range of 3-10 dB larger than, a sound level detected via a sensor of the sleep or respiratory appliance.

In a still further embodiment, operating the sleep or respiratory appliance further includes determining sleep quality parameters based on (i) the generated quantitative diagnostic snoring information and (ii) sensor data from the sleep or respiratory appliance. The sleep quality parameters may include apnea, hypopnea and or AHI (Apnea Hypopnea Index). The generated quantitative diagnostic snoring information may include one or more of snoring level, frequency spectrum, pressure amplitude, timing of snoring, and number of snoring events. The sensor data from the sleep or respiratory appliance can include at least flow rate data.

In yet another embodiment, operating the sleep or respiratory appliance can further include adjusting a characteristic of the delivery of the pressurized flow of breathable gas based on at least the detected snoring and the sleep quality parameters. The adjusted characteristic of the delivery of the pressurized flow of breathable gas can include changing one or more of a mask pressure and a humidifier setting, i.e., for a sleep appliance equipped with a humidifier.

In one embodiment, a continuous positive airway pressure CPAP sleep appliance with snore detection comprises a blower, a patient interface device, one or more sensors, a communications module, and a controller adapted to operate the sleep appliance for delivery of a pressurized flow of breathable gas to the patient interface device. The blower is configured to provide the pressurized flow of breathable gas. The patient interface device is configured for being worn by a user. The patient interface device is further for being fluidly coupled to the blower via a patient circuit for delivery of the pressurized flow of breathable gas from the blower to the patient interface device. The one or more sensors are configured to acquire (i) sensor information for determination of (i)(a) a moment or start of inspiration of the user or (i)(b) a moment or start of expiration of the user during the delivery of the pressurized flow of breathable gas.

The communications module is adapted for wirelessly communicating with an app on a mobile device. When executed, the app on a mobile device is configured to: (i) record, via a microphone of the mobile device, acoustic signals during a period of use of the sleep appliance by the user, (ii) analyze, via a processor of at least one of the mobile device, the sleep appliance, or a remote cloud device, the recorded acoustic signals for generating processed audio data that is relevant to detect snoring, and (iii) link, via a communication means, at least one or more of the recorded acoustic signals, the analyzed acoustic signals, and the recognized snoring characteristics or events between (a) at least one of the mobile device or the remote cloud device, and (b) the sleep appliance. In one embodiment, analyzing the recorded acoustic signals includes at least using an acoustic model configured to recognize snoring characteristics or events by inspecting transient acoustic pressure levels and/or a frequency spectrum in the recorded acoustic signals. In addition, operating the sleep appliance can include snoring detection based on a combination of (i) sensor information obtained from sensors of the sleep appliance and (ii) the processed audio data which is used to detect snoring, and wherein operating the sleep appliance can further include generating quantitative diagnostic snoring information.

In another embodiment, snoring detection may include combining, via the app alone, the sleep appliance alone, the remote cloud device alone, or a combination of the app, the sleep appliance, and the remote cloud device (i) the sensor information obtained from sensors of the sleep or respiratory appliance and (ii) data handling of processed audio data from the mobile device. With respect to sensor information, the sensor information is obtained from sensors of the sleep or respiratory appliance for determination of (i)(a) a moment or start of inspiration or (i)(b) a moment or start of expiration. With respect to data handling, the processed audio data from the mobile device is for use in (ii)(a) detecting a noise spectrum at a start of a sleep procedure that includes providing the flow of breathable gas to the patient interface device, prior to or before a user begins snoring and (ii)(b) detecting snoring via a sound level in the processed audio data above a snoring sound minimum level threshold and one or more of the moments or starts of inspiration and expiration.

In another embodiment, the sleep appliance comprises wherein generating quantitative diagnostic snoring information is based on the detected snoring. The quantitative diagnostic snoring information comprises an improved snoring information over similar snoring information based on use of the sleep appliance alone. In yet another embodiment, snoring detection further comprises time synchronization of the recorded acoustic signals from the mobile device with the sleep appliance sensor information relating the recorded acoustic information with phase information of a breathing cycle of the user.

In a further embodiment, the sleep appliance includes wherein snoring detection further comprises (i) detecting (a) a moment (i.e., time of occurrence) of rise in a level of sound captured or recorded via the microphone of the mobile device (i.e., processed audio data) above a snoring sound minimum level threshold (also above the background noise) that correlates with (i.e., correlating with, matching, or equaling) either one or a combination of both (b)(1) the moment of inhalation (i.e., determined via sleep appliance sensor signals signaling inhalation) and (b)(2) the moment of exhalation (i.e., determined via sleep appliance sensor signals signaling exhalation) within a snoring sound detection time threshold, and (ii) otherwise not detecting. In one embodiment, the snoring sound detection time threshold comprises within a time of ± 0.4 seconds. Determining the moment of rise in the level of sound captured or recorded via the microphone of the mobile device (i.e., processed audio data) includes background noise filtering by subtracting a background noise level of the noise spectrum prior to determining the rise above the snoring sound minimum level threshold. The background noise level of the noise spectrum may be due to the sleep appliance, determined from the level of sound captured or recorded via the microphone of the mobile device (i.e., in the processed audio data).

In accordance with another embodiment, the app is initiated via at least one of (i) a user input on the mobile device to start the app, and (ii) a sleep appliance routine. The sleep appliance routine is configured to wirelessly search and connect with the mobile device, wherein, responsive to being wirelessly connected with the mobile device, the sleep appliance routine (a) automatically starts the app or (b) causes a user inquiry requesting user input on the mobile device for confirmation to start the app. In yet another embodiment, a sound level threshold of the recorded acoustic signals via the microphone of the mobile device is in a range of 5-20 dB larger than, or in a range of 3-10 dB larger than, a sound level detected via a sensor of the sleep or respiratory appliance.

In a still further embodiment, operating the sleep appliance further includes determining sleep quality parameters based on (i) the generated quantitative diagnostic snoring information and (ii) sensor data from the sleep appliance. The sleep quality parameters may include apnea, hypopnea and or AHI (Apnea Hypopnea Index). The generated quantitative diagnostic snoring information may include one or more of snoring level, frequency spectrum, pressure amplitude, timing of snoring, and number of snoring events. The sensor data from the sleep appliance can include at least flow rate data.

In yet another embodiment, operating the sleep appliance can further include adjusting a characteristic of the delivery of the pressurized flow of breathable gas based on at least the detected snoring and the sleep quality parameters. The adjusted characteristic of the delivery of the pressurized flow of breathable gas can include changing one or more of a mask pressure and a humidifier setting, i.e., for a sleep appliance equipped with a humidifier.

The embodiments of the present disclosure advantageously solve the problem of inefficiencies in snore detection of current sleep or respiratory appliances. Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing Figures, like reference numerals refer to like elements. In addition, it is to be noted that the Figures may not be drawn to scale.
Fig. 1 is a block diagram view of sleep appliance (e.g., a continuous positive airway pressure (CPAP) device) with snore detection and a mobile device featuring an app, according to an embodiment of the present disclosure;
Fig. 2 is a graphical representation view of illustrative sensor signals vs. time for use in determining a moment or start of inspiration of the user or a moment or start of expiration of the user during delivery of a pressurized flow of breathable gas, according to an embodiment of the present disclosure; and
Fig. 3 is a flow diagram view of a method for snore detection in a sleep or respiratory appliance (e.g., a continuous positive airway pressure CPAP device) according to further embodiments of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

Nowadays there are very good microphones in modern mobile telephones. These microphones are much more sensitive to acoustic pressure waves than the pressure sensor in a typical sleep or respiratory appliance. Accordingly, the inventors recognize that such microphones could be used to detect snoring during night, by analyzing the measured noise signal captured by the microphone of the mobile phone. The description herein makes reference to a mobile device, which can include a mobile phone, tablet device, smart watch or other mobile device of similar characteristics. In addition, the inventors recognize that, via Bluetooth or Wi-Fi or other means known in the art, a communication connection can be made between the mobile device and the sleep or respiratory appliance.

In addition, the inventors discovered that it is possible to detect snoring by means of an acoustic model (e.g., similar to automatic speech recognition (ASR)) that recognizes snoring by inspecting transient acoustic pressure levels and or the frequency spectrum recorded by the microphone of the mobile device, especially in the frequency range from 10 to 3000 Hz and more specifically in the range of 50 to 300 Hz.

When the measured signal by the mobile device is linked with the sleep or respiratory appliance audio sensor output, it is possible to detect whether the user of the appliance or a partner is producing the snoring. In addition, snoring sound will be different during inhalation and exhalation. Especially during inhalation, the snoring sound will be large. The moment of inhalation during a breathing cycle can be easily detected by the flow sensor only or in combination with the pressure sensor in the sleep or respiratory appliance or device. Other options for determining the moment of inhalation may also be possible. In this way, it is possible to easily determine who is snoring and at which level.

With the communication connection, several parameters describing the snoring can be stored. For example, snoring parameters may include: snoring level, frequency spectrum, pressure amplitude, timing of snoring, number of snoring events, etc. The information obtained regarding the determined snoring parameters can be used advantageously to adjust the settings of the sleep or respiratory appliance, e.g., at a later stage, preferably by medical trained staff.

It is also possible that the information obtained regarding the determined snoring parameters can be used advantageously to adjust the sleep or respiratory appliance settings during sleep. This could be possible in CPAP, BiPAP, or autoPAP mode. Especially, the mask pressure could be adjusted based on determined snoring parameters.

According to one embodiment, the method of snore detection includes the use of an app, which a user of the sleep appliance would download on his or her mobile device. Through the use of the app, information of the mobile device can also be used by the sleep or respiratory appliance. A communication connection can be made between the mobile device and the sleep or respiratory appliance, for example, via Bluetooth^{™}, Wi-Fi, or other means known in the art.

In one embodiment the sound level variation as detected by the microphone of the mobile device is linked with sensor output from the sleep or respiratory appliance, i.e., at least flow sensor information. Especially, a rapid change (or increase) in flow rate delivered by the ventilator or blower of the sleep or respiratory appliance is a sign of inhalation. Other sensor output from the sleep or respiratory appliance can also be used, such as a change in RPM of the blower motor or a change in current or pressure, or combinations of these sensor outputs. In this way, it is possible to link the inhalation period with a sound level of the microphone of the mobile device. In the same way, a change in sound level as recorded by the microphone of the mobile device during exhalation is another sign of snoring by the user of the sleep or respiratory appliance. The exhalation period can be determined in a similar way as inhalation and standard methods for determining inhalation or exhalation can be used. Further it is also possible to use the mode of operation, e.g., CPAP, BiPAP, autoPAP, etc. to link the snoring sounds to the inhalation or exhalation information.

In one embodiment, the method includes performing an audio frequency analysis on the recorded microphone signals of the mobile device via software on the mobile device. In another embodiment, the method includes performing frequency analysis of the recorded mobile device microphone signals in the sleep or respiratory appliance. In addition, the method includes performing snoring detection by inspection of the sound level in a particular frequency domain (range or band), for example, in the 10 to 3000 Hz range and, more particularly, in the 25 to 500 Hz range and, even more preferably, in the 50 to 150 Hz range. When the amplitude as recorded by the microphone of the mobile device is exceeding a certain threshold during the inhalation phase of the breathing pattern, this is a sign or indication of snoring. In particular, when the amplitude in the snoring frequency band (or range) is reducing during the exhalation phase of the breathing cycle and the amplitude in another frequency range is increasing due to the exhalation, this also is an indication of snoring.

According to one embodiment, the snoring amplitude threshold is set such that it distinguishes between snoring and the normal operation of the sleep or respiratory appliance, as well as potential background noise. Therefore, the snoring amplitude threshold is preferably linked to the pressure setting of the sleep or respiratory appliance and preferably combined with the maximum flow rate of breathable gas going through the sleep or respiratory appliance.

As the recorded sound level by sleep or respiratory appliance is much lower than the sound level generated by mildly snoring persons, it will be easy to detect already mildly snoring. Preferably a snoring amplitude threshold is in the range of 5-20 dB larger than the sound level produced by the sleep or respiratory appliance, more preferably, in the range of 3-10 dB larger than the sound level produced by the sleep or respiratory appliance.

According to one embodiment, the distance of the mobile device to the user of the sleep or respiratory appliance is preferably within one meter. The sound spectrum recorded by the mobile device may be used as a means of determining the distance between the mobile device and the sleep or respiratory appliance, and in this way know the distance between the user and the sleep or respiratory appliance within approximately 1 meter. In particular, part of the frequency spectrum that is close to n times the rotational frequency of the fan or motor of the blower can be used to estimate the distance as the sound produced in this frequency range by the sleep or respiratory appliance may give a large tone.

In one embodiment, all recorded audio information may be stored. Preferably, only audio information that exceeds the threshold is stored. In addition, a number of parameters describing the snoring (also referred to herein as snoring parameters) can be stored. Snoring parameters may include, but are not limited to: snoring level, frequency spectrum, pressure amplitude, timing of snoring, number of snoring events, etc. The information provided by the snoring parameters can be used to obtain information relating to a wellbeing of a patient and/or used to adjust the settings of the sleep or respiratory appliance in a later stage, preferably by medical trained staff. In another embodiment, the method utilizes the information provided by the snoring parameters describing the snoring of the user to adjust the sleep or respiratory appliance settings during sleep. Such an adjustment of the sleep or respiratory appliance could be possible in CPAP, BiPaP, or autoPaP mode. For instance, in response to predetermined information ascertained via one or more snoring parameters, the sleep or respiratory appliance is configured to adjust the mask pressure.

In one example, it could be possible that not only the user of the sleep appliance, but also the bed partner, is producing snoring sound or even the bed partner only. According to one embodiment of the snoring detection method described herein, the microphone of the mobile device in combination with the coupling to the sleep or respiratory appliance can be configured to distinguish between the user and the bed partner. In this embodiment, the method includes linking the snore detection to the breathing phase of the user measured by the sleep or respiratory appliance. In addition, the snoring sound can be stored and recognized by software configured for snore recognition in case both user and bed partner are producing snoring sounds. For example, upon recognition, the snoring sound from the bed partner can be subtracted from the total sound recorded by the microphone of the mobile device in order to remove the bed partner snoring sound. Subtracting the snoring sound from the bed partner can be done multiple times. When the snoring sound by the bed partner changes, all potential snoring sounds can be used to improve the snoring signal, i.e., of the user of the sleep appliance, to be analyzed. The embodiments of the present disclosure are applicable in sleep appliances, e.g., like CPAP, BiPAP, and other similar appliances, etc.

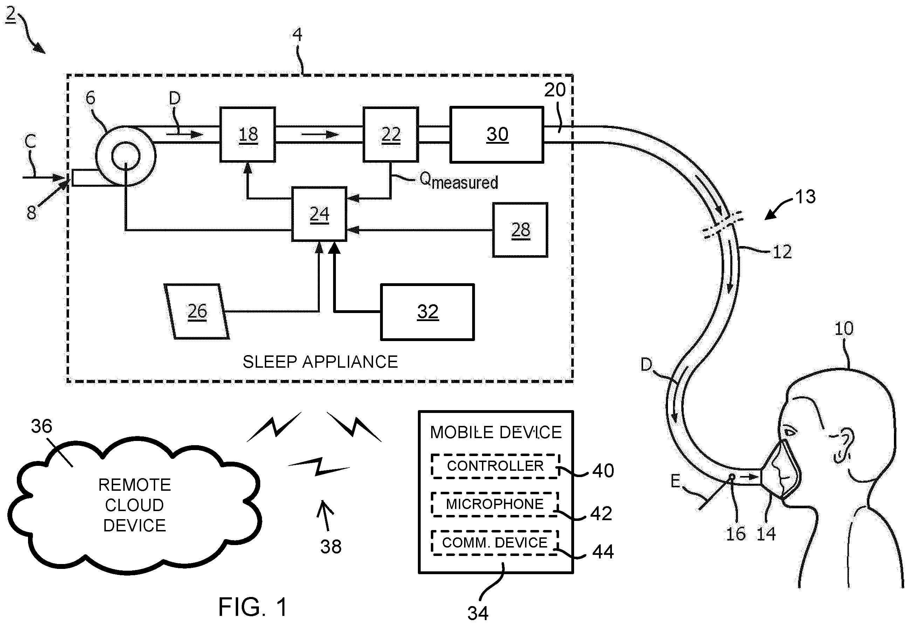

With reference now to Fig. 1, an example of a sleep or respiratory appliance system 2 is shown which may be employed as a portion of one particular, non-limiting exemplary embodiment of the present invention. Sleep or respiratory appliance system 2 includes a pressure support device 4 which houses a blower assembly 6, an example of which will be described in further detail below. Blower assembly 6 receives breathing gas, generally indicated by arrow C, from the ambient atmosphere through a filtered air inlet 8 provided as part of pressure support device 4, and generates a flow of breathing gas therefrom for delivery to an airway of a patient 10 at relatively higher and lower pressures, i.e., generally equal to or above ambient atmospheric pressure, to generate pressure to provide pressure compensation to patient 10 via a patient circuit which includes a delivery conduit 12 and a patient interface device 14. In the exemplary embodiment, blower assembly 6 is capable of providing a flow of breathing gas ranging in pressure from 2-30 cm H₂O. The pressurized flow of breathing gas from blower assembly 6, generally indicated by arrow D, is delivered via the delivery conduit 12 to the breathing mask or patient interface device 14 of any known construction, which is typically worn by or otherwise attached to patient 10 to communicate the flow of breathing gas to the airway of patient 10. Delivery conduit 12 and patient interface device 14 are typically collectively referred to as the patient circuit.

Sleep or respiratory appliance system 2 shown in Fig. 1 is what is known as a single-limb system, meaning that the patient circuit includes only delivery conduit 12 connecting patient 10 to Sleep or respiratory appliance system 2. As such, an exhaust vent 16 is provided in delivery conduit 12 for venting exhaled gases from the system as indicated by arrow E. It should be noted that exhaust vent 16 can be provided at other locations in addition to or instead of in delivery conduit 12, such as in patient interface device 14. It should also be understood that exhaust vent 16 can have a wide variety of configurations depending on the desired manner in which gas is to be vented from sleep or respiratory appliance system 2.

The present concept also contemplates that sleep or respiratory appliance system 2 can be a two-limb system, having a delivery conduit and an exhaust conduit connected to patient 10. In a two-limb system (also referred to as a dual-limb system), the exhaust conduit carries exhaust gas from patient 10 and includes an exhaust valve at the end distal from patient 10. The exhaust valve in such an embodiment is typically actively controlled to maintain a desired level or pressure in the system, which is commonly known as positive end expiratory pressure (PEEP).

Furthermore, in the illustrated exemplary embodiment shown in Fig. 1, patient interface device 14 comprises a nasal/oral mask. It is to be understood, however, that patient interface device 14 can include a nasal mask, nasal pillows, a tracheal tube, an endotracheal tube, or any other device that provides a suitable gas flow communicating function. Also, for purposes of the present invention, the phrase "patient interface" can include delivery conduit 12 and any other structures that couple the source of pressurized breathing gas to patient 10.

In the illustrated embodiment, sleep or respiratory appliance system 2 includes a pressure controller in the form of a valve 18 provided in internal delivery conduit 20 provided in a housing of pressure support device 4. Valve 18 controls the pressure of the flow of breathing gas from blower assembly 6 that is delivered to patient 10. For present purposes, blower assembly 6 and valve 18 are collectively referred to as a pressure generating system because they act in concert to generate and control the pressure and/or flow of gas delivered to patient 10. However, it should be apparent that other techniques for controlling the pressure of the gas delivered to patient 10, such as varying the speed of blower assembly 6, either alone or in combination with a pressure control valve, are contemplated by the present invention. Thus, valve 18 is optional depending on the technique used to control the pressure of the flow of breathing gas delivered to patient 10. If valve 18 is eliminated, the pressure generating system corresponds to blower assembly 6 alone, and the pressure of gas in the patient circuit is controlled, for example, by controlling the speed of blower assembly 6.

Sleep or respiratory appliance system 2 further includes a flow sensor 22 that measures the flow of the breathing gas within delivery conduit 20 and delivery conduit 12. In the particular embodiment shown in Fig. 1, flow sensor 22 is interposed in line with delivery conduits 20 and 12, most preferably downstream of valve 18. Sleep or respiratory appliance system 2 additionally includes a pressure sensor 28 that detects the pressure of the pressurized fluid in delivery conduit 20. While the point at which the flow is measured by flow sensor 22 and the pressure is measured by pressure sensor 28 are illustrated as being within pressure support device 4, it is to be understood that the location at which the actual flow and pressure measurements are taken may be anywhere along delivery conduits 20 or 12. The flow of breathing gas measured by flow sensor 22 and the pressure detected by pressure sensor 28 are provided to a processing unit 24 to determine the flow of gas at patient 10 (Q_{PATIENT}). In one embodiment, sleep appliance 4 includes one or more sensors configured to acquire (i) sensor information for determination of (i)(a) a moment or start of inspiration of the user or (i)(b) a moment or start of expiration of the user during the delivery of the pressurized flow of breathable gas, as will be discussed further herein.

Referring still to Fig. 1, processing unit 24 includes a processing portion which may be, for example, a microprocessor, a microcontroller or some other suitable processing device, and a memory portion that may be internal to the processing portion or operatively coupled to the processing portion and that provides a storage medium for data and software executable by the processing portion for controlling the operation of sleep or respiratory appliance system 2. Processing unit 24 is structured to receive outputs of one or more sensors, such as those previously discussed, which are structured to gather data related to effectiveness of the pressure support therapy. Processing unit 24 is also structured to analyze outputs of the sensors while pressure support therapy is provided to the patient to determine patient airflow and pressure waveforms in the patient circuit. An input/output device 26 is provided for setting various parameters used by sleep or respiratory appliance system 2, as well as for displaying and outputting information and data to a user, such as a clinician or caregiver. Pressure support device 4 may also include a humidifier 30 for humidifying the flow of breathable gas to the patient interface device, according to the requirements of a given sleep or respiratory appliance implementation.

In addition, sleep or respirator appliance system 2 and/or pressure support device 4 includes a communication means 32, which is coupled to processing unit 24, for performing various communications between devices, as discussed herein. Communication means 32 can comprise, for example, any suitable means for communicating between the pressure support device 4 and a mobile device 34 and/or a remote cloud device 36 for snoring detection according to the embodiments of the present disclosure. In Fig. 1, the lightning bolts identified via reference numeral 38 are indicative of a wired or wireless link (e.g., via near field communications, Bluetooth^{™}, Wi-Fi, or other wireless communications link) between a given component (e.g., controller 24) of the system 2 and the mobile device 34 and/or the remote cloud device 36. Preferably, communication means 32 comprises a means for wirelessly communicating with mobile device 34 and/or remote cloud device 36.

Mobile device 34 can comprise a mobile phone, smart phone, smart watch, tablet, iPad^{™}, or any other suitable mobile electronic device, configured to operate in a manner according to the embodiments of the present disclosure. In one embodiment, mobile device 34 comprises a controller or processor 40, a microphone 42, and a communication device 44. During usage of the sleep or respiratory appliance 4, a snore detection app on the mobile device 34 is executed via the controller or processor 40. The snore detection app is configured to:(i) record, via the microphone 42 of the mobile device 34, acoustic signals during a period of use of the sleep appliance 2 by the user. The app is further configured to (ii) analyze, via the processor 42 of the mobile device 34, the recorded acoustic signals for generating processed audio data that is relevant to detect snoring. Analyzing the recorded acoustic signals includes at least using an acoustic model that recognizes snoring characteristics or events by inspecting transient acoustic pressure levels and/or a frequency spectrum in the recorded acoustic signals. Furthermore, the app is configured to (iii) link, via the communication means 44, at least one or more of the recorded acoustic signals, the analyzed acoustic signals, and the recognized snoring characteristics or events between the mobile device and the sleep appliance 4. In addition, remote cloud device 36 comprises a device separate from the sleep appliance 4 and the mobile device 34. The remote cloud device 36 comprises at least a cloud-based device having capabilities similar to those of the mobile device 34.

In one embodiment, in connection with the acoustic model mentioned herein above, the controller or processor 40 may include a machine learning unit, i.e., collection of information to determine trends and probability of snoring. In accordance with one embodiment, the machine learning unit can comprise a machine learning model trained using the data collected by all sensors (e.g., but not limited to, flow, pressure, environmental parameter, demographic, etc.). The snore detection acoustic model or algorithm can rely on supervised learning such as classification or regression-based methods (support vector machine/SVM, Linear discriminant analysis/LDA, logistic regression, etc.), unsupervised learning such as clustering and dimensionality reduction methods, or deep learning such as deep neural networks, recurrent neural networks, convolutional neural networks, GANs or similar. The machine learning unit can output probability of snoring or specific snoring trends.

Similar to existing pressure support devices, processing unit 24 is programmed to utilize sensors (e.g., flow sensor 22, pressure sensor 28) within pressure support device 4 to summarize sleep quality (e.g., sleep disordered breathing events) of the patient when pressure support device 24 is in use by the patient. Unlike existing arrangements, processing unit 24 is further programmed to receive and analyze supplemental data from one or more of a mobile device 34 or a remote cloud device 36 for snoring detection, as will be discussed herein.

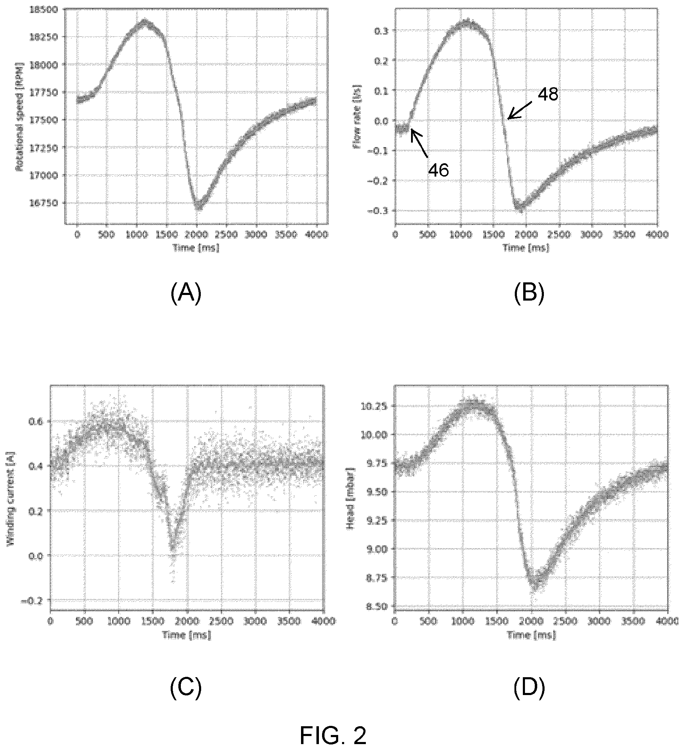

Turning now to Fig. 2, there is shown a graphical representation view of illustrative sensor signals vs. time for use in determining a moment or start of inspiration of the user or a moment or start of expiration of the user during delivery of a pressurized flow of breathable gas, according to an embodiment of the present disclosure. In particular, information obtained via sensors available in the sleep or respiratory appliance 4 can be used in the process of distinguishing patient snoring from background noise. An experimental artificial lung set-up or a breathing simulator was used to simulate respiratory conditions on a ventilator in a given mode of ventilation to generate data. For example, the interactive control settings included: breath rate (15 BPM); resistance trachea (10 cm H₂O/L/s); compliance (80 mL/cm H₂O); flex setting (OFF); and pressure setting (10 Cm H₂O). Experiments were executed without a mask or patient interface device, so the sleep or respirator appliance was directly coupled to the artificial lung set-up. Normally, a mask or patient interface device with leakage valve included would be applied, which would shift the flow rate, RPM and current, but the shape of the graphical representations or curves would remain similar. Fig. 2(A) is a plot of rotational speed (in RPM) versus time (in milli-seconds). Fig. 2(B) is a plot of flow rate (in 1/s) versus time (in milli-seconds). Fig. 2(C) is a plot of winding current (in amps) versus time (in milli-seconds). Lastly, Fig. 2(D) is a plot of head pressure (in mbar) versus time (in milli-seconds).

With reference to Fig. 2(B), a positive flow rate is indicative of an inhalation, and a negative flow rate is indicative of an exhalation. From the plots in Fig. 2, it can be clearly observed that a correlation can be seen between the flow rate pattern, the RPM, the current or the pressure pattern. The start of an inhalation (indicated by reference numeral 46 in Fig. 2(B)) is announced as the moment where the flow rate (corrected with the leakage flow through the valve, where the flow rate through the valve can be easily predicted from the mask pressure) is almost zero (preferably in the range of ± 0.1l/s and more preferable in the range of ± 0.05 l/s) combined with a large (almost discontinuous) positive change of the derivative of the flow rate with time. Here the change in flow rate derivative is between 0.05 l/s² to 1 l/s² and more preferably in the range of 0.1 to 0.6 l/s². The start of an expiration (indicated by reference numeral 48 in Fig. 2(B)), which can be another moment in the change of the patient snoring signal, can be detected from the flow rate signal (corrected with the flow rate through the valve in the mask) as the moment where the flow rate is zero, but now with large negative change of the derivative of the flow rate with time (with a more or less constant negative derivative in flow rate around the moment of the start of the expiration).

From the sensor data it can be shown that the curves for RPM, pressure, current are comparable to the flow rate curve. Hence, instead of flow rate data, also data from RPM and/or pressure and or current could be used to detect the moment of inspiration and expiration of the patient. The start of an inspiration can be detected from a sudden positive change in dRPM/dt, and or dΔp/dt and/or dI/dt. Preferably the absolute value at the moment of the sudden positive change of all sensor values is stored as well, as this can be used to determine the start of the expiration. To determine the gradients in flow rate, RPM, I (current) or Δp (pressure) all known methods in the art can be used, such as averaging over multiple data points etc. The start of the expiration can be obtained from the point where the absolute values of the sensor reading are equal to the values for the start of the inspiration, however here we have a large negative derivative in dRPM/dt, and or dΔp/dt and/or dI/dt. The moment of inspiration or expiration based on the RPM and/or Δp and/or I data could be improved by taking a weighted average of the inspiration and expiration moments based on the individual sensors only.

For the data handling, there are several options. The raw data information (captured or recorded audio data) from the microphone of the mobile device is processed (via an initial processing step) on the mobile device via the App, where the sampling rate is sufficiently high, larger than 1 kHz and more preferably larger than 8 kHz. The processing of the data (via a subsequent processing step) i.e. band frequency filtering or other known filtering techniques, such as anti-aliasing, (smoothing of the data), etc., can be done either (a) on the mobile device (via the App) or (b) on the sleep or respiratory appliance device. Similarly, the processing of the data could also be done on the remote cloud device. In one embodiment, the combination of the sensor information from the sleep or respiratory appliance device and the processed audio data from the mobile device can be either done in the mobile device (i.e., requires wireless data transport from sleep or respiratory appliance device to mobile device) or on the sleep or respiratory appliance device (i.e., requires wireless data transport from the mobile device to the sleep or respiratory appliance device). Preferably, this combination of data handling and drawing conclusions on the moment of snoring of a patient is done on the sleep or respiratory appliance device, as potential change of the settings of the sleep or respiratory appliance device is then easier, but it is also possible to combine the data on the mobile device, via the app and then send the combined data to the sleep or respiratory appliance device. Note that both a mask pressure and or humidifier settings (for implementations that include a humidifier) could be changed based on the snore detection.

In one embodiment, the data of the snoring detection is preferably combined with flow rate data to determine apnea, hypopnea and or AHI (Apnea Hypopnea Index). In one embodiment, data collection and band frequency filtering is performed via the mobile device, and combined data handling and drawing conclusions regarding the moment of snoring is done on the sleep or respiratory appliance device.

In another embodiment, machine learning could be implemented via the app to use previous snoring data of the patient to easily distinguish patient snoring from noise sources. Further the App can also store a noise spectrum when the sleep appliance is switched on. This is the moment where the patient is not yet snoring, but the background noise level of the sleep appliance, which may vary with mask pressure level, could be easily detected. This background noise level due to the sleep appliance can be subtracted from microphone signals of the mobile device and/or of the sleep appliance to detect snoring.

According to another embodiment, the communication between the mobile device and the sleep appliance device could be according the standard wireless communication protocols. Via the app, the user registers and connects with the sleep or respiratory appliance device. At the start of the sleeping procedure, the user could start the app via the mobile device and/or it may also be possible that the sleep or respiratory appliance device searches contact with the mobile device and starts the app automatically and/or asks the user whether he or she wants to start the app on the mobile device.

Snore detection and especially filtering from background noise (i.e., the background noise level) can be done to compare the moment of rise of the sound (preferably in a specific frequency domain) above a background sound level (where correction with the sound produced by the sleep appliance is possible) correlates with the sleep or respiratory appliance device sensor signals signaling inhalation within a time of ± 0.4 seconds, and more preferable within ± 0.2 seconds. A further improvement is possible where either a sudden change in or increase in sound level is possible just after an exhalation detection, in a similar way as an inhalation. The effects in sound level with respect to both inhalation and exhalation could also be combined for snore detection.

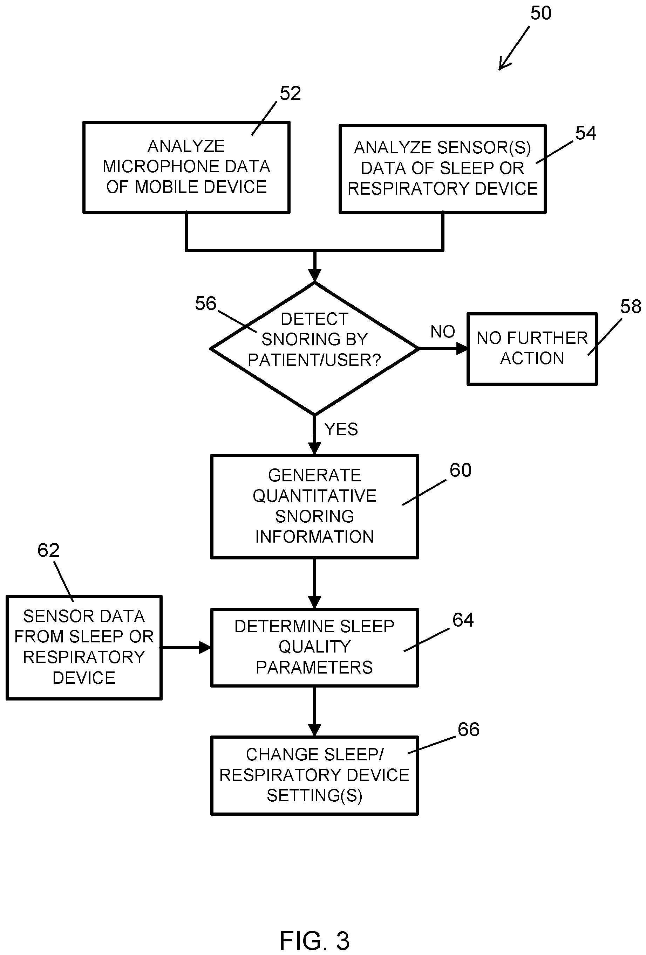

With reference now to Fig. 3, a flow diagram view of a method 50 for snore detection in a sleep or respiratory appliance (e.g., a continuous positive airway pressure CPAP device) according to further embodiments of the present disclosure is shown. In Step 52, audio data captured via the microphone of the mobile device is recorded and analyzed. Concurrently, in Step 54, sensor data is captured via the sleep or respiratory appliance device is captured and analyzed. In Step 56, the analyzed microphone data from the mobile device and the sensor data of the sleep or respiratory appliance device are processed to determine whether snoring by the patient/user is detected. If no snoring is detected, then the method proceeds to Step 58, in which there is no further action taken. Alternatively, the method may return to steps 52 and 54 and repeat itself for the duration of sleep appliance usage over a period of sleep. In Step 56, in response to detection of snoring by the patient/user, the method proceeds to Step 60.

In Step 60, quantitative snoring information is generated based on at least the audio data captured by the microphone of the mobile device. The method continues at Step 64 with the determination of sleep quality parameters. The determination of sleep quality parameters is based on the quantitative snoring information generated in Step 60 and sensor data obtained from the sleep or respiratory appliance device (Step 62). In a further step (Step 66), the sleep or respiratory appliance device is configured to change one or more device settings based on at least the determined sleep quality parameters. For example, the sleep or respiratory appliance device may be configured to adjust the mask pressure or a humidifier setting.

As disclosed herein, controller 24 of the sleep appliance device 4 and controller 40 of the mobile device 34 may comprise one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given snoring detection implementation and/or application, as disclosed herein. Controller 24 or 40 can further comprise one or more of various modules. It is understood that method as disclosed herein may be implemented in modules, wherein the modules may be computer program modules which are rendered in a non-transitory computer-readable medium.

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be advantageously used in any number of sleep appliances, such as, CPAP, BiPAP, and other similar appliances. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A method (50) for snore detection in a sleep or respiratory appliance (4), comprising:
executing an app on a mobile device (34), the app configured to:
(i) record, via a microphone (42) of the mobile device (34), acoustic signals during a period of use of the sleep or respiratory appliance (4) by a user (10) during a period of sleep,
(ii) analyze (52,54), via a processor (24,42) of at least one of the mobile device (34), the sleep or respiratory appliance (4), or a remote cloud device (36), the recorded acoustic signals for generating processed audio data to detect snoring, wherein analyzing the recorded acoustic signals includes at least using an acoustic model configured to recognize snoring characteristics or events by inspecting transient acoustic pressure levels and/or a frequency spectrum in the recorded acoustic signals, and
(iii) link, via a communication means (32,44), at least one or more of the recorded acoustic signals, the analyzed acoustic signals, and the recognized snoring characteristics or events between (a) at least one of the mobile device (34) or the remote cloud device (36), and (b) the sleep or respiratory appliance (4); and
operating the sleep or respiratory appliance (4) for delivery of a pressurized flow of breathable gas (D) to a patient interface device (14) to be worn by a user (10) of the sleep or respiratory appliance (4), wherein operating includes snoring detection (56) based on a combination of (i) sensor information obtained from sensors (22,28) of the sleep or respiratory appliance (4) and (ii) the processed audio data which is used to detect snoring, and wherein operating further includes generating (60) quantitative diagnostic snoring information.

2. The method (50) according to claim 1, wherein snoring detection (56) includes combining, via the app alone, the sleep or respiratory appliance (4) alone, the remote cloud device (36) alone, or a combination of the app, the sleep or respiratory appliance, and the remote cloud device (i) the sensor information obtained from sensors (22,28) of the sleep or respiratory appliance (4) for determination of (i)(a) a moment or start of inspiration or (i)(b) a moment or start of expiration, and (ii) data handling of processed audio data from the mobile device (34) (ii)(a) for use in detecting a noise spectrum at a start of a sleep procedure that includes providing the flow of breathable gas (D) to the patient interface device (14), prior to or before a user (10) begins snoring and (ii)(b) for use in detecting snoring via a sound level in the processed audio data above a snoring sound minimum level threshold and one or more of the moments or starts of inspiration and expiration.

3. The method (50) according to claim 1 or 2, wherein generating (60) diagnostic quantitative snoring information is based on the detected snoring, wherein the quantitative diagnostic snoring information comprises an improved snoring information over similar snoring information based on use of the sleep or respiratory appliance alone.

4. The method (50) according to any of the preceding claims, wherein snoring detection (56) further comprises time synchronization of the recorded acoustic signals from the mobile device (34) with the sleep or respiratory appliance (4) sensor information relating the recorded acoustic information with phase information of a breathing cycle of the user (10).

5. The method (50) according to any of the preceding claims, wherein snoring detection (56) further comprises (i) detecting (a) a moment of rise in a level of sound captured or recorded via the microphone (42) of the mobile device (34) above a snoring sound minimum level threshold that correlates with either one or a combination of both (b)(1) the moment of inhalation and (b)(2) the moment of exhalation within a snoring sound detection time threshold, and (ii) otherwise not detecting.

6. The method (50) according to claim 5, wherein determining the moment of rise in the level of sound captured or recorded via the microphone (42) of the mobile device (34) includes background noise filtering by subtracting a background noise level of the noise spectrum prior to determining the rise above the snoring sound minimum level threshold.

7. The method (50) according to any of the preceding claims, wherein the app is initiated via at least one of (i) a user input on the mobile device (34) to start the app, and (ii) a sleep appliance routine configured to wirelessly search and connect with the mobile device (34), wherein, responsive to being wirelessly connected with the mobile device (34), the sleep appliance routine (a) automatically starts the app or (b) causes a user inquiry requesting user input on the mobile device (34) for confirmation to start the app.

8. The method (50) according to any of the preceding claims, wherein a sound level threshold of the recorded acoustic signals via the microphone (42) of the mobile device (4) is in a range of 5-20 dB larger than, or in a range of 3-10 dB larger than, a sound level detected via a sensor of the sleep or respiratory appliance (4).

9. The method (50) according to any of the preceding claims, wherein operating (66) the sleep or respiratory appliance (4) further includes determining (64) sleep quality parameters based on (i) the generated quantitative diagnostic snoring information and (ii) sensor data from the sleep or respiratory appliance (4) wherein the sleep quality parameters include apnea, hypopnea and or AHI (Apnea Hypopnea Index), wherein the generated quantitative diagnostic snoring information (60) includes one or more of snoring level, frequency spectrum, pressure amplitude, timing of snoring, and number of snoring events, and wherein the sensor data from the sleep or respiratory appliance (4) includes at least flow rate data.

10. The method (50) according to any of the preceding claims, wherein operating (66) further includes adjusting a characteristic of the delivery of the pressurized flow of breathable gas based on at least the detected snoring and the sleep quality parameters.

11. A continuous positive airway pressure CPAP sleep appliance (4) with snore detection, comprising:
a blower (6) configured to provide a pressurized flow of breathable gas (D);
a patient interface device (14) configured for being worn by a user (10), the patient interface device further being fluidly coupled to the blower via a patient circuit (12) for delivery of the pressurized flow of breathable gas from the blower to the patient interface device;
one or more sensors (22,28) configured to acquire (i) sensor information for determination of (i)(a) a moment or start of inspiration of the user or (i)(b) a moment or start of expiration of the user during the delivery of the pressurized flow of breathable gas;
a communications module (32) adapted for wirelessly communicating with an app on a mobile device (34) that, when executed, is configured to:
(i) record, via a microphone (42) of the mobile device (34), acoustic signals during a period of use of the sleep appliance (4) by the user (10),
(ii) analyze, via a processor (24,42) of at least one of the mobile device (34), the sleep appliance (4), or a remote cloud device (36), the recorded acoustic signals for generating processed audio data that is relevant to detect snoring, wherein analyzing the recorded acoustic signals includes at least using an acoustic model configured to recognize snoring characteristics or events by inspecting transient acoustic pressure levels and/or a frequency spectrum in the recorded acoustic signals, and
(iii) link, via a communication means (32,44), at least one or more of the recorded acoustic signals, the analyzed acoustic signals, and the recognized snoring characteristics or events between (a) at least one of the mobile device (34) or the remote cloud device (36), and (b) the sleep appliance (4); and
a controller (24) adapted to operate the sleep appliance (4) for delivery of the pressurized flow of breathable gas (D) to the patient interface device (14), wherein operating includes snoring detection based on a combination of (i) sensor information obtained from sensors (22,28) of the sleep appliance (4) and (ii) the processed audio data which is used to detect snoring, and wherein operating further includes generating diagnostic quantitative snoring information.

12. The sleep appliance (4) according to claim 11, wherein snoring detection includes combining, via the app alone, the sleep appliance (4) alone, the remote cloud device (36) alone, or a combination of the app, the sleep appliance, and the remote cloud device (i) the sensor information obtained from sensors (22,28) of the sleep or respiratory appliance (4) for determination of (i)(a) a moment or start of inspiration or (i)(b) a moment or start of expiration, and (ii) data handling of processed audio data from the mobile device (34) (ii)(a) for use in detecting a noise spectrum at a start of a sleep procedure that includes providing the flow of breathable gas (D) to the patient interface device (14), prior to or before a user (10) begins snoring and (ii)(b) for use in detecting snoring via a sound level in the processed audio data above a snoring sound minimum level threshold and one or more of the moments or starts of inspiration and expiration.

13. The sleep appliance (4) according to any one of claim 11-12, wherein generating diagnostic quantitative snoring information is based on the detected snoring, wherein the diagnostic quantitative snoring information comprises an improved snoring information over similar snoring information based on use of the sleep appliance alone.

14. The sleep appliance (4) according to any one of claim 11-13, wherein snoring detection further comprises time synchronization of the recorded acoustic signals from the mobile device (34) with the sleep appliance sensor information relating the recorded acoustic information with phase information of a breathing cycle of the user (10).

15. The sleep appliance (4) according to any one of claim 11-14, wherein snoring detection further comprises (i) detecting (a) a moment of rise in a level of sound captured or recorded via the microphone (42) of the mobile device (34) above a snoring sound minimum level threshold that correlates with either one or a combination of both (b)(1) the moment of inhalation and (b)(2) the moment of exhalation within a snoring sound detection time threshold, and (ii) otherwise not detecting.
